# EUROPÄISCHE PATENTSCHRIFT

(11) **EP 0 717 609 B1**
(45) Veröffentlichungstag und Bekanntmachung des Hinweises auf die Patenterteilung: **07.04.1999**
(21) Anmeldenummer: 94927573.9
(22) Anmeldetag: 07.09.1994
(51) Int. Cl.: A61F 2/42

(54) **GELENKPROTHESE FÜR KLEINE GELENKE**
PROSTHESIS FOR SMALL JOINTS
PROTHESE POUR PETITES ARTICULATIONS

(30) Priorität: 07.09.1993 DE 4330248
(43) Veröffentlichungstag der Anmeldung: 26.06.1996
(73) Patentinhaber: Copf, Franz, D-70178 Stuttgart (DE)
(72) Erfinder: COPF, Franz, D-70178 Stuttgart (DE); RENTSCH, Gunter, D-70174 Stuttgart (DE); REILL, Peter, D-72076 Tübingen (DE); HERMAN, Srecko, 6100 Lubljana (SI)
(74) Vertreter: Ostertag, Reinhard
(86) Internationale Anmeldenummer: EP9402977
(87) Internationale Veröffentlichungsnummer: WO9507060

(56) Entgegenhaltungen:
- EP-A- 0 245 846
- EP-A- 0 328 848
- WO-A-89/06946
- WO-A-93/00053
- DE-A- 2 839 150
- DE-A- 3 536 895
- DE-A- 3 613 657
- DE-B- 2 338 137
- FR-A- 2 425 237
- GB-A- 2 056 862
- US-A- 4 204 284
- US-A- 5 147 386

## Beschreibung

Die Erfindung betrifft eine Gelenkprothese gemäß dem Oberbegriff des Anspruches 1.

Eine derartige Gelenkprothese ist in der EP 0 245 846 B1 beschrieben, wobei auch eine Verwendung als Fingergelenksprothese erwähnt ist. In der Praxis werden derartige Prothesen aber nur für Hüftgelenke verwendet.

Für kleine Gelenke, wie Finger- oder Zehengelenke finden dagegen derzeit in der Praxis ausschließlich Schaftprothesen Verwendung, die unter Verwendung von Zement auf Kunststoffbasis nach Resezieren des alten Gelenkes in die Enden der Finger- bzw. Zehenknochen einzementiert werden. Derartige einzementierte Gelenkprothesen für kleine Gelenke haben jedoch nur eine verhältnismäßig kurze Lebensdauer, da sich die Schäfte der Prothesenteile wieder von den Knochenwänden ablösen.

Durch die vorliegende Erfindung soll daher eine Gelenkprothese der eingangs beschriebenen Art so weitergebildet werden, daß sie sich speziell für den Einsatz an kleinen Gelenken wie Finger- oder Zehengelenken eignet.

Diese Aufgabe ist erfindungsgemäß gelöst durch eine Gelenkprothese mit den in Anspruch 1 angegebenen Merkmalen.

Bei den gegenüber dem Oberschenkelknochen bzw. dem Beckenknochen nur geringe Abmessung aufweisenden Knochen der Hand und des Fußes hat man für ein zementfreies Implantieren nur ein verhältnismäßig kleines Spongiosavolumen zur Verfügung. Die Erfinder haben herausgefunden, daß man trotzdem eine zementfreie Implantation von Prothesenteilen in diesen kleinen Durchmesser aufweisenden Röhrenknochen vornehmen kann, wenn man dafür Sorge trägt, daß die verschiedenen Verankerungselemente sich nahe bei der harten Corticalis befinden. Auf diese Weise kann man auf die Verankerungseinheit ausgeübte Kippmomente gut aufnehmen und es steht für die Spongiosa insgesamt viel freier Raum zur Verfügung, um nach Regeneration ein tragfähiges Spongiosagerüst zu bilden. Um eine solche Anordnung trotz der von Person zu Person wechselnden Knochengeometrien gewährleisten zu können, wird erfindungsgemäß vorgeschlagen, die Verankerungselemente zumindest in ihrem Endabschnitt elastisch oder plastisch verformbar auszubilden. Auf diese Weise erhält man eine automatische Formanpassung der Verankerungseinheit an den jeweils mit einem Prothesenteil zu versehenden Knochen, wobei große Abmessungsunterschiede, wie bei Prothesen allgemein üblich, durch Verankerungseinheiten unterschiedlicher Größe aufgenommen werden.

Vorteilhafte Weiterbildungen der Erfindungen sind in Unteransprüchen angegeben.

Mit der Weiterbildung der Erfindung gemäß Anspruch 2 erhält man lange biegefähige Abschnitte der Verankerungselemente und eine unabhängige Einstellung der Verankerungselemente auf die jeweilige Knochengeometrie.

Hierbei kann man gemäß Anspruch 3 die Steifheit der Verankerungselemente in unmittelbarer Nachbarschaft der Tragplatte erhöhen und in demjenigen Bereich, in welchem der mit Spongiosa erfüllte Raum des Knochens noch größere Abmessungen hat, zusätzliche Verankerungsflächen zwischen Spongiosa und Verankerungseinheit bereitstellen.

Eine Gelenkprothese, wie sie im Anspruch 4 angegeben ist, hat eine Verankerungseinheit mit besonders einfacher Geometrie. Diese läßt sich somit sehr kostengünstig herstellen.

Hierbei wird mit der Weiterbildung der Erfindung gemäß Anspruch 5 erreicht, daß die Berührflächen, über welche die Verankerungseinheit und das Spongiosamaterial lokal zusammenarbeiten, verhältnismäßig klein sind. Auf diese Weise erhält man eine lokale Reizung der Spongiosa, die im Hinblick auf gute Durchblutung und laufende gute Regeneration von Vorteil ist.

Die Weiterbildung der Erfindung gemäß Anspruch 6 ist wiederum in Hinblick auf gute Formanpassung der Verankerungseinheit an die jeweilige Knochengeometrie von Vorteil.

Bildet man die Gelenkprothese gemäß Anspruch 7 aus, so hat man eine große Anzahl von kleinen Berührflächen zwischen der Verankerungseinheit und dem Spongiosamaterial, was im Hinblick auf gute Verankerung und gute dynamische Regeneration der Spongiosa von Vorteil ist.

Die Weiterbildung der Erfindung gemäß Anspruch 8 ist hierbei im Hinblick auf einfache Herstellung der Gießformen für die Verankerungseinheit und im Hinblick auf ein einfaches Einführen der Verankerungseinheit in das ausgeräumte Knochenende von Vorteil.

Die Weiterbildung der Erfindung gemäß Anspruch 9 gewährleistet eine gleichförmige Einleitung von Kräften in die verschiedenen Abschnitte des Spongiosamateriales. Darüber hinaus bilden die verschiedenen sekundären Verankerungselemente zugleich eine Art Käfig, welcher gemahlenes Spongiosamaterial etwas festhalten kann, wenn dieses in die Verankerungseinheit eingefüllt in den ausgeräumten Endabschnitt des Knochen eingeführt wird.

Die Weiterbildung der Erfindung gemäß Anspruch 10 sorgt für eine nochmals gleichförmigere Verteilung der Krafteinleitung in die Spongiosa.

Mit der Weiterbildung der Erfindung gemäß Anspruch 11 ist gewährleistet, daß die Verankerungseinheit nach dem Einführen in das ausgeräumte Knochenende im leichten Pressitz dort vorläufig gehalten wird, was die Arbeit des Operateurs erleichtert.

Dabei ist gemäß Anspruch 12 gewährleistet, daß das Einsetzen des Prothesenteiles unter elastischer und/oder plastischer Verformung der Verankerungselemente ohne große Kraftaufwendung und ohne Verhaken der Verankerungsscheiben an der Corticalis erfolgen kann.

Sieht man gemäß Anspruch 13 zusätzliche angeformte ballige Kontaktkörper vor, so kann man im übrigen die Verankerungselemente durchgehend als zylindrichen Kern mit aufgesetzen Verankerungsscheiben ausbilden.

Bei einer Prothese gemäß Anspruch 14 bilden von radial nach außen über die Sollbegrenzungsfläche überstehenden Verankerungscheibenabschnitten freie Abschnitte der Verankerungselemente zugleich auch die Kontaktflächen, über welche die Verankerungseinheit mit der Corticalis in Berührung steht.

Um auch in den den Kontaktflächen benachbarten Abschnitten der Verankerungselemente eine Verankerungswirkung mit der Spongiosa zu erhalten, kann man dort Verankerungsscheiben vorsehen, wie sie im Anspruch 15 angegeben sind.

Die Abmessungen für die Verankerungsscheiben und Kerne der Verankerungselemente, wie sie in den Ansprüchen 16-18 angegeben sind, sind im Hinblick auf eine ausreichende Reizung der Spongiosa bei den im täglichen Gebrauch des Gelenkes auftretenden Belastungen einerseits und im Hinblick auf das Vermeiden unzulässig großer Belastungen der Spongiosa von Vorteil.

Die Weiterbildung der Erfindung gemäß Anspruch 19 ist im Hinblick auf ein einfaches Befestigen der aus Kunststoffmaterial hergestellten Gelenkelemente an den aus Metall bestehenden Tragplatten von Vorteil.

Bei einer Prothese, wie sie im Anspruch 20 angegeben ist, hat man eine zwangsläufige Begrenzung, des Drehwinkels des zweiachsigen Kugelgelenkes in beliebigen Arbeitsrichtungen. Durch Randkonturierung der Anschlagscheibe kann man eine für unterschiedliche Arbeitsrichtungen unterschiedliche Winkelbegrenzung und Anschlagwirkung einfach vorgeben, falls gewünscht.

Diese Winkelbegrenzung erfolgt bei einer Prothese gemäß Anspruch 21 elastisch, wobei man mit der Weiterbildung der Erfindung gemäß Anspruch 22 einen weiteren Parameter für die Vorgabe der Anschlag-Federwirkung hat, nämlich die Abmessungen der radialen Schlitze, die zur Dicke des freiauskragenden Randabschnittes hinzukommen.

Ein Prothesenteil gemäß Anspruch 23 oder 25 eignet sich besonders zur Verwendung an solchen Gelenken, die auch auf Zug belastet werden. Ein derartiges Gelenk mit in Gelenkachse gegeneinander bewegbaren Gelenkelementen stellt nach Implantierung auch insoweit ein gutes Imitat eines natürlichen Gelenkes dar, als bei Zugbelastung zunächst die das Gelenk umgebenden Bänder beansprucht werden, bevor dann die Kraftübertragung zunehmend und ggf. uberwiegend über die Gelenkelemente erfolgt.

Gemäß Anspruch 24 kann man bei einem solchen primär einachsigen Gelenk zusätzlich eine kleine Verkippbarkeit in zur Hauptachse senkrechter Richtung gewährleisten.

Nachstehend wird die Erfindung anhand von Ausführungsbeispielen unter Bezugnahme auf die Zeichnung näher erläutert. In dieser zeigen:
- Figur 1:: einen Längsschnitt durch eine erste Gelenkprothese für Finger- oder Zehengelenke;
- Figur 2:: einen Längsschnitt durch eine zweite Gelenkprothese für kleine Gelenke;
- Figur 3:: einen Längsschnitt durch eine Verankerungseinheit für eine weiter abgewandelte Gelenkprothese für kleine Gelenke;
- Figur 4:: eine ähnliche Ansicht wie Figur 2, in welcher jedoch eine Gelenkprothese mit Wälzgelenk wiedergegeben ist; und
- Figur 5:: einen axialen Schnitt durch eine nochmals abgewandelte Gelenkprothese mit einem Biegegelenk.

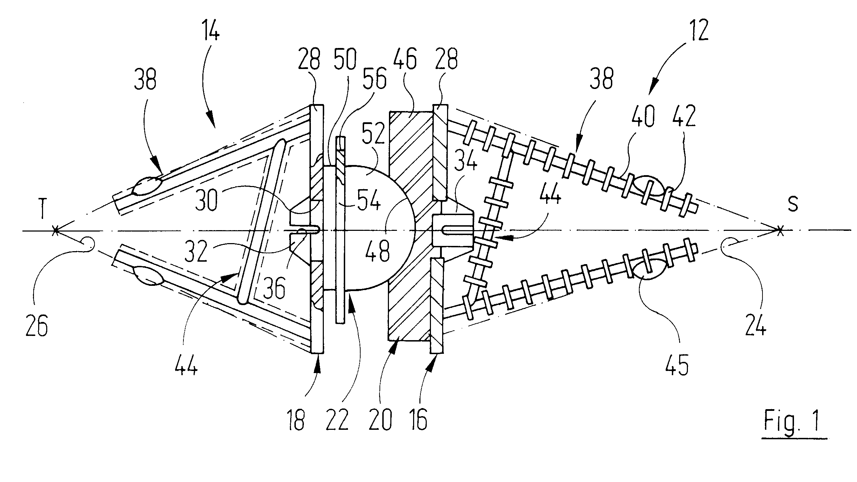

In Figur 1 ist eine Gelenkprothese für ein Mittelhandfingergelenk wiedergegeben, die aus zwei insgesamt mit 12 bzw. 14 bezeichneten Prothesenteilen besteht.

Die Prothesenteile 12, 14 bestehen jeweils aus einer mit 16 bzw. 18 bezeichneten Verankerungseinheit und einem mit dieser verrasteten Gelenkelement 20 bzw. 22.

Die Verankerungseinheiten 16, 18 haben im wesentlichen gleichen Aufbau, unterscheiden sich nur geringfügig in ihrer Geometrie, welche an die jeweiligen Knochenenden angepaßt ist, in welchen das betrachtete Prothesenteil anzubringen ist. Für die Zwecke der vorliegenden Beschreibung sei angenommen, daß in den Knochenenden jeweils eine im wesentlichen kegelförmige Aufnahme durch Ausräumen von Spongiosamaterial erzeugt wird. Die idealisierten Begrenzungswände dieser Aufnahmeöffnungen sind bei 24 bzw. 26 strichpunktiert angedeutet. Für die Zwecke der Beschreibung sei angenommen, daß die Aufnahmen 24, 26 beim freien Ende im wesentlichen gleichen Durchmesser haben, sich jedoch in der Tiefe unterscheiden (Lage der Kegelspitze S bzw. T).

Die Verankerungseinheiten 16, 18 haben jeweils eine Tragplatte 28 mit einer mittigen Öffnung 30, durch welche ein Befestigungszapfen 32 bzw. 34 des zugeordneten Gelenkelementes 20, 22 unter elastischem Zusammendrükken einführbar ist. Wie aus der Zeichnung ersichtlich, haben die Befestigungszapfen 32, 34 jeweils eine Mehrzahl in Umfangsrichtung verteilter axialer Schlitze 36 und sind in ihrem über die Rückseite (vom Gelenk abliegende Seite) der Tragplatte 28 vorspringenden Abschnitt kegelförmig ausgebildet, wobei ein äußerster Rand des Kegels radial über den Rand der Öffnung 30 übersteht.

An die Tragplatten 28 sind jeweils mehrere stabförmige Verankerungselemente 38 angegossen, die jeweils einen stabförmigen Kernabschnitt 40 und auf den letzteren aufgesetzte axial beabstandete Verankerungsscheiben 42 aufweisen. In der Praxis haben die Kernabschnitte 40 einen Durchmesser von etwa 0,8-1,5 mm, die Verankerungsscheiben 42 einen Durchmesser, der um etwa 0,6-1,5 mm größer ist als der Durchmesser des Kernabschnittes 40. Der axiale Abstand der Verankerungsscheiben 42 beträgt in der Praxis etwa 2-4 mm, ihre Dicke etwa 0,6-1,2 mm.

Beim betrachteten Ausführungsbeispiel sind vier Verankerungselemente 38 vorgesehen, deren Fußpunkte im wesentlichen ein Rechteck aufspannen (bei ovalem Knochenquerschnitt). In ihrem der Tragplatte 28 benachbarten Abschnitten sind die Verankerungselemente 38 durch Versteifungselemente 44 verbunden, die ihrerseits ebenfalls wieder einen Kernabschnitt und von diesem getragen Verankerungsscheiben aufweisen, wie für die Verankerungselemente 38 beschrieben. Beim hier betrachteten Ausführungsbeispiel sind nur jeweils zwei der Verankerungselemente 38 durch Versteifungselemente 44 verbunden. In Abwandlung kann man zusätzlich auch senkrecht zur Zeichenebene von Figur 1 verlaufende Versteifungselemente vorsehen, wenn man auch in dieser Richtung eine Versteifung der Verankerungseinheit in der Nachbarschaft der Tragplatte 28 wünscht.

Bei den freien Enden sind an die Außenseiten der Verankerungselemente 38 linsenförmige, ballige Kontaktkörper 45 angegossen, deren äußere Kalotten über die Verankerungsscheiben 42 überstehen und so ein leichtes Einschieben der Verankerungseinheit über die nach Ausräumen verbleibende Knochenwand ermöglichen, ohne daß die Verankerungsscheiben dort festhaken, so daß Späne abgestossen werden müssten, um die Verankerungselemente weiter vorzuschieben; denn wie aus der Zeichnung ersichtlich, liegt die Begrenzungsfläche der Aufnahme 24 bzw. 26 teilweise radial innerhalb der Außenkontur der zugeordneten Verankerungseinheit 16 bzw. 18.

Die Verankerungseinheiten 16, 18 sind einstückige Gußteile aus einem gewebeverträglichen Teil bzw. einer gewebeverträglichen Metallegierung. Vorzugsweise sind die Verankerungseinheiten 16, 18 aus Titan gegossen.

Die Gelenkelemente 20, 22 sind Spritzteile aus gewebeverträglichem Kunststoffmaterial, insbesondere Polyäthylen.

Das Gelenkelement 20 hat eine den Befestigungszapfen 32 tragende Grundplatte 46, in welcher eine sphärische Gelenkpfanne 48 ausgebildet ist. Das Gelenkelement 22 hat eine Grundplatte 50, deren Rückseite den Befestigungszapfen 34 und deren Vorderseite eine sphärische Gelenkkugel 52 trägt. Beim Fuß der Gelenkkugel 52 ist eine radial auskragende Anschlagscheibe 54 an die Gelenkkugel 52 angeformt, die in ihrem Randbereich zusätzlich mit radialen Schlitzen 56 versehen sein kann, um im äußersten Randbereich die Federwirkung, die in der Anschlagscheibe 54 an sich schon aufgrund der Materialwahl und der Scheibendicke erhalten wird, herabzusetzen.

Wie aus Figur 1 ersichtlich, ist die Tragplatte 28 der Verankerungseinheit 16 nicht exakt senkrecht zur Achse des Prothesenteiles 12 ausgerichtet, vielmehr unter einem kleinen Winkel w aus der exakt transversalen Orientierung herausgekippt. Dies ist für ein Mittelhandfingergelenk von Vorteil.

Bei implantierter Prothese schließen die Tragplatten 28 der Prothesenteile 12, 14 die Enden der zu verbindenden Knochen bündig ab, d.h. die Umfangsflächen der Tragplatten werden noch von der bis zur Tragplattenstirnfläche reichenden Cortikalis des ausgeräumten und wieder mit Knochenmehl verfüllten Knochenendes überdeckt.

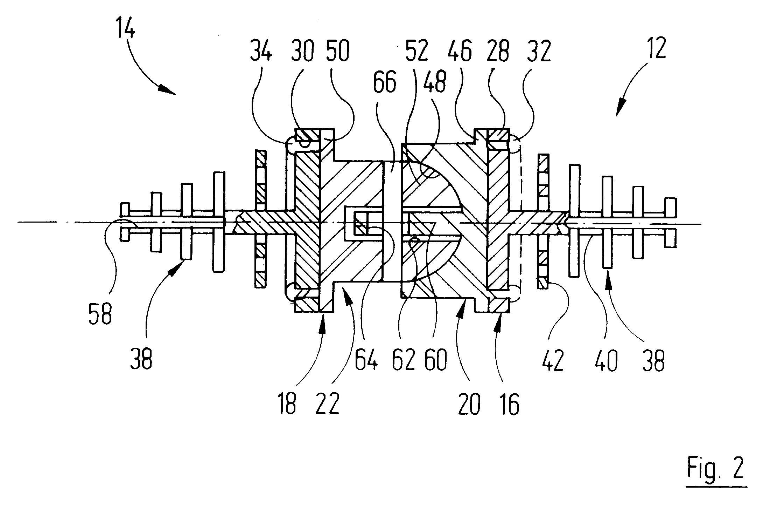

Bei der abgewandelten Gelenkprothese nach Figur 2 sind Prothesenteile, die funktionsmäßig unter Bezugnahme auf Figur 1 schon erläuterten Prothesenteilen entsprechen, wieder mit denselben Bezugszeichen versehen.

An die Tragplatten 28 ist jetzt jedoch nur jeweils ein einziges Verankerungselement 38 mittig angeformt, welches größeren Durchmesser aufweist und eine Mehrzahl größeren Durchmesser aufweisender Verankerungsscheiben 42 trägt. Die Verankerungsscheiben 42 haben jeweils Gitterstruktur, wobei die Gitterstege so gewählt sind, daß man beim Gebrauch des Gelenkes eine vergleichbare Flächenpressung von gegen die Verankerungsscheiben 42 gewachsenem Spongiosamaterial erhält, wie bei den Verankerungseinheiten nach Figur 1.

In den axial außenliegenden Abschnitten weisen die Verankerungselemente 38 und die Verankerungsscheiben 42 vier in Umfangsrichtung verteilte Schlitze 58 auf, so daß die Sektoren der Verankerungsscheiben 42 unabhängig voneinander radial einfedern können.

Die Gelenkelemente 20, 22 sind nunmehr über eine Mehrzahl in Umfangsrichtung am Rand verteilter Befestigungszapfen 32, 34 mit den zugeordneten Tragplatten 28 verbunden, wobei die Befestigungszapfen 32, 34 zunächst reine Stabform hatten und ihre Köpfe durch Formen des thermoplastischen Materiales unter Erwärmung nach Ansetzen der Gelenkelemente an die Tragplatte erhalten wurden.

In weiterer Abwandlung gegenüber Figur 1 ist das Gelenk als im wesentlichen einachsiges Gelenk ausgebildet, das heißt die zusammenarbeitenden Lagerflächen von Gelenkpfanne 48 und Gelenkkugel 52 sind Zylinderflächen. Von der Gelenkpfanne 48 steht ein Steg 60 vor, der unter seitlichem Spiel einen Schlitz 52 der Gelenkkugel 52 eingreift. Der Steg 60 ist mit einem Langloch 64 ausgebildet, durch welches sich ein in der Gelenkkugel 52 festgelegter Stift 66 erstreckt.

Bei dem in Figur 2 gezeigten Gelenk arbeiten die Lagerflächen von Gelenkpfanne und Gelenkkugel nur dann zusammen, wenn das Gelenk auf Druck beansprucht ist. Wird das Gelenk dagegen auf Zug beansprucht, so werden diese beiden Lagerflächen aufgrund der Stift/Langloch-Verbindung 64, 66 voneinander abgehoben und die Kraftübertragung vom Gelenkelement 22 auf das Gelenkelement 20 erfolgt über die Stift/Langloch-Verbindung. Bei Zugbelastung ist dann auch das Gelenkelement 22 bezüglich des Gelenkelementes 20 in zur Hauptachse des Gelenkes senkrechter Richtung geringfügig verkippbar, so wie dies das Spiel zwischen dem Steg 60 und dem Schlitz 62 zuläßt. Ein derartiges Gelenk eignet sich insbesondere als Mittelfingergelenk.

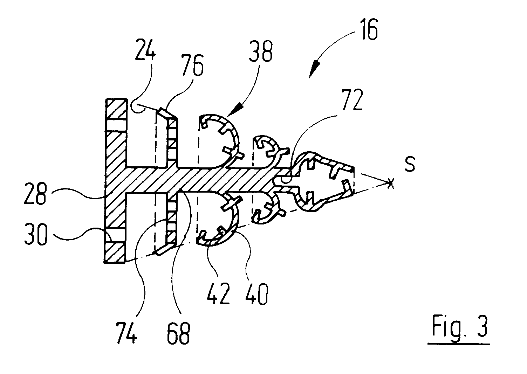

Bei der in Figur 3 gezeigten abgewandelten Verankerungseinheit sind bei einem zentralen, größeren Durchmesser aufweisenden Hauptverankerungselement 68, das an die Rückseite der Tragplatte 28 mittig angeformt ist, im in Figur 3 rechts gelegenen Abschnitt mehrere Gruppen von sekundären Verankerungselementen 38 angeformt, die jeweils kreisbogenförmig sind. Der Erstreckungswinkel dieser Kreisbogen beträgt etwa 200°. In denjenigen Abschnitten, in welchen die Verankerungselemente 38 über die Aufnahme 24 überstehen, sind sie von radial überstehenden Abschnitten der Verankerungsscheiben 42 frei. Diese können dort entweder als halbe Scheiben oder vorzugsweise als exzentrische, nur nach innen verlaufende Scheiben ausgebildet sein.

Die Verankerungselemente 38 einer in einer gemeinsamen transversalen Ebene liegenden Gruppe sind in Umfangsrichtung unter gleichem Winkelabstand angeordnet. Die Verankerungselemente 38 axial benachbarter Gruppen sind in der Zeichnung der besseren Darstellbarkeit halber als in gleichen axialen Ebenen liegend wiedergegeben. Bei praktischen Ausführungsbeispielen werden dagegen die Verankerungselemente 38 benachbarter Gruppen in Umfangsrichtung versetzt, vorzugsweise um eine halbe Teilung, damit man möglichst gleichförmige spongiosaerfüllte Volumina hat.

Wo Verankerungselemente 38 unter kleinem radialem Abstand voneinander verlaufen (rechter Endabschnitt der Verankerungseinheit), sind die Verankerungsscheiben 42 zusätzlich axial gegeneinander versetzt, um ein radiales Einfedern der Verankerungselemente 38 nicht zu behindern. Außerdem ist der Endabschnitt des Tragstabes 58 mit einem Schlitz 72 versehen, der ebenfalls das Einfedern der endständigen Verankerungselemente 38 begünstigt.

In unmittelbarer Nachbarschaft der Tragplatte 28 hat die in Figur 3 gezeigte Verankerungseinheit eine großen Durchmesser aufweisende Verankerungsscheibe 74, die wieder mit einer großen Anzahl von Durchbrechungen versehen ist und somit eine gitterähnliche Struktur hat. Randabschnitte der Verankerungsscheibe 74 sind mit radialen Schlitzen 76 versehen, und diese Randabschnitte sind vorzugsweise wieder gekrümmt, um eine Formanpassung an die im Knochen erzeugte Aufnahme zu ermöglichen und das Einführen in die unterdimensionierte Aufnahme 24 zu erleichtern.

Aus Figur 3 ist ersichtlich, daß die Verankerungsscheibe 74 und Verankerungselemente 38 zusammen eine Art Käfig bilden, in welchem gemahlenes Spongiosamaterial zurückgehalten wird, so daß dieses zusammen mit der Verankerungseinheit in die im Knochen erzeugte Aufnahme eingeführt werden kann.

Auch die Verankerungseinheit nach Figur 3 ist ein einstückiges Gußteil aus Titan oder einer gewebeverträglichen Metallegierung.

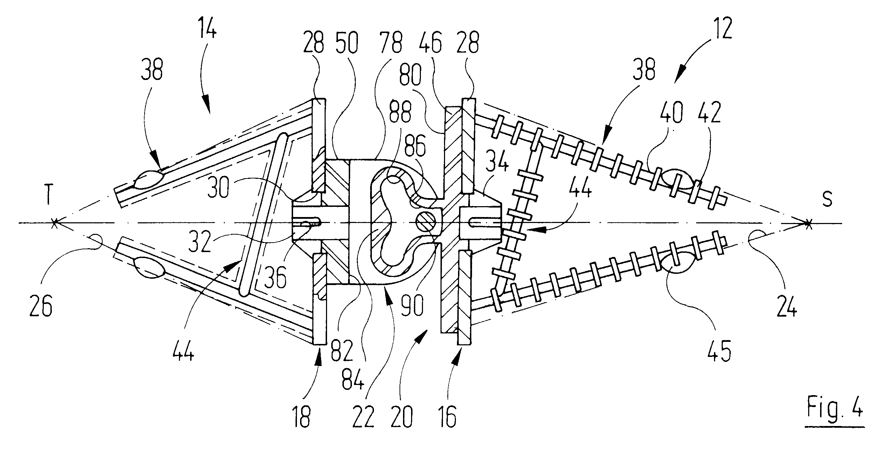

Bei der Gelenkprothese nach Figur 4 sind wiederum schon beschriebene Bauelemente mit den schon oben verwendeten Bezugszeichen versehen.

An der Grundplatte 50 des Gelenkelementes 22 ist ein zylindrisches Wälzelement 78 angeformt, dessen zylindrische Rollfläche auf einer Wälzfläche 80 läuft, die durch die Stirnfläche der Grundplatte 46 des Gelenkelementes 20 gebildet ist.

Das Wälzelement 78 hat einen zur Wälzachse senkrechten Schlitz 82, in welchem eine Lasche 84 unter Spiel läuft, die an die Grundplatte 46 des Prothesenteiles 12 angeformt ist. Die Lasche 84 hat eine einem Knochenende ähnliche Geometrie und begrenzt einen Führungsschlitz mit einem senkrecht zur Wälzfläche 80 verlaufenden Schlitzabschnitt 86 sowie einem hierzu im wesentlichen transversalen Schlitzabschnitt 88. Die Ränder der Schlitzabschnitte 86, 88 entsprechen im wesentlichen einer Epizykloide.

In den Schlitzabschnitten 86, 88 läuft ein aus der Achse des Wälzelementes 78 zur Wälzfläche 80 hin versetzter Führungsstift 90, der vom Wälzelement 78 getragen ist. Der Durchmesser des Führungsstiftes 90 ist etwas kleiner als die Breite der Schlitzabschnitte 86, 88, das Spiel des Führungsstiftes in der Schlitzanordnung in zur Wälzfläche 80 senkrechter Richtung beträgt für ein Fingergelenk etwa 1,5 bis 2 mm.

Ist die in Figur 4 gezeigte Prothese auf Druck belastet, so rollt das Wälzelement 78 ohne Gleiten auf der Wälzfläche 80 ab, wobei der Führungsstift 90 den Rändern der Schlitzabschnitte 86, 88 folgt. Wird die in Figur 4 gezeigte Prothese etwas zugentlastet oder werden bei nur kleiner Druckbelastung große Drehmomente auf sie ausgeübt, so kann die Umfangsfläche des Wälzelementes 78 auf der Wälzfläche 80 auch durchrutschen. Schließlich läßt sich bei stärkerer Zugbelastung des Gelenkes das Wälzelement 78 von der Wälzfläche 80 ganz abheben, so daß eine leichte Drehbarkeit der Prothesenteile 12, 14 gegeneinander gewährleistet ist.

Bei implantiertem Gelenk wird das Wälzelement 78 unter der Kraft der das Gelenk umgebenden Sehnen und Bänder in Anlage an der Wälzfläche 80 gehalten.

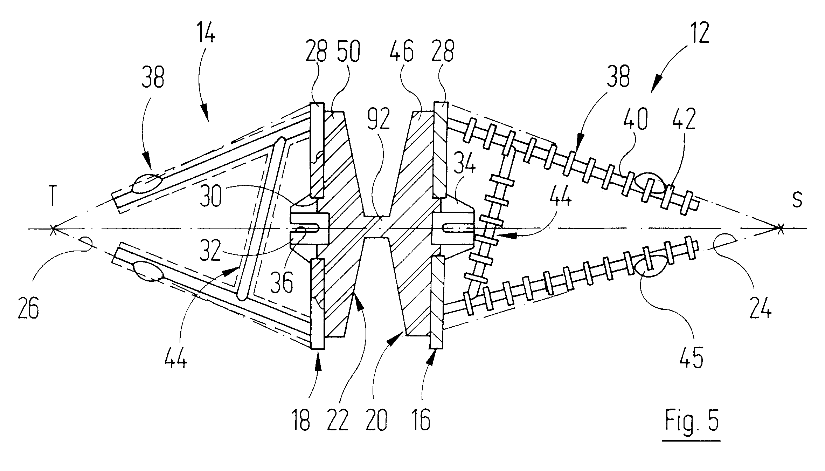

Bei der in Figur 5 gezeigten Prothese sind auf den Tragplatten 28 der Prothesenteile 12, 14 zwei abgeschrägte Grundplatten 46, 50 angebracht, die durch einen angeformten Biegeabschnitt 92 verbunden sind. Ist dieser Biegeabschnitt 92 stabförmig, läßt sich das Gelenk in beliebiger Richtung biegen, hat der Biegeabschnitt 92 in zur Zeichenebene senkrechter Richtung rechteckigen Querschnitt, so hat man ein im wesentlichen einachsiges Scharniergelenk.

## Patentansprüche

1. Gelenkprothese für kleine Gelenke wie Finger- oder Zehengelenke, mit zwei Prothesenteilen (12, 14), die jeweils eine Verankerungseinheit (16, 18) und ein von dieser getragenes Gelenkelement (20, 22) aufweisen, wobei die Verankerungseinheiten (16, 18) jeweils eine Tragplatte (28) und mindestens ein Verankerungselement (38) aufweisen, welches eine Mehrzahl in axialer Richtung aufeinanderfolgender radialer Verankerungsscheiben (42) trägt, dadurch gekennzeichnet, daß die Verankerungselemente (38) zusammen eine im wesentlichen kegelförmige Außenkontur aufweisen und zumindest in einem Endabschnitt elastisch und/oder plastisch verformbar sind, so daß sie sich beim Einsetzen in ein Knochenende an die Knochengeometrie automatisch anpassen.

2. Gelenkprothese nach Anspruch 1, dadurch gekennzeichnet, daß die Verankerungselemente (38) im wesentlichen stabförmig sind und jeweils für sich an der Tragplatte (28) angebracht sind.

3. Gelenkprothese nach Anspruch 2, dadurch gekennzeichnet, daß die Verankerungselemente (38) in der Nachbarschaft der Tragplatte (28) durch Versteifungselemente (44) verbunden sind, welche ebenfalls eine Mehrzahl in axialer Richtung aufeinanderfolgender radialer Verankerungsscheiben (42) tragen.

4. Gelenkprothese nach Anspruch 1 oder 2, dadurch gekennzeichnet, daß ein einziges Verankerungselement (38) im wesentlichen mittig an der Tragplatte (28) angebracht ist, daß die Verankerungsscheiben (42) ausgehend von einem in der Nachbarschaft der Tragplatte (28) mit deren Durchmesser vergleichbaren Durchmesser einen zum freien Ende des Verankerungselementes (38) hin zunehmend kleineren Durchmesser haben und daß in einem Endabschnitt des Verankerungselementes (38) die Verankerungsscheiben (42) axiale Schlitze (58) aufweisen.

5. Gelenkprothese nach Anspruch 4, dadurch gekennzeichnet, daß die Verankerungsscheiben (42) als Gitter ausgebildet sind.

6. Gelenkprothese nach Anspruch 4 oder 5, dadurch gekennzeichnet, daß die radial außenliegenden Abschnitte der Verankerungsscheiben (42) mit Schlitzen (76) versehen sind und radial verformbar sind.

7. Gelenkprothese nach einem der Ansprüche 1, 2 oder 4, dadurch gekennzeichnet, daß an einem im wesentlichen mittig an der Tragplatte (28) befestigtem Hauptverankerungselement (68) sekundäre Verankerungselemente (38) angebracht sind, welche zumindest in ihren Endabschnitten elastisch und/oder plastisch verformbar sind und jeweils eine Mehrzahl axial beabstandeter, verglichen mit dem Durchmesser der Tragplatte (28) kleinen Durchmesser aufweisender Verankerungsscheiben (42) tragen.

8. Gelenkprothese nach Anspruch 7, dadurch gekennzeichnet, daß die sekundären Verankerungselemente (38) jeweils im wesentlichen kreisbogenförmig sind, wobei ihre Umfangserstreckung mehr als 180°, vorzugsweise jedoch weniger als 225° beträgt und die offene Seite des Kreisbogens zur Tragplatte (28) weist.

9. Gelenkprothese nach Anspruch 7 oder 8, dadurch gekennzeichnet, daß die sekundären Verankerungselemente (38) in axial beabstandeten Gruppen angeordnet sind, wobei die in einer gemeinsamen radialen Ebene liegenden sekundären Verankerungselemente (38) einer Gruppe in Umfangsrichtung unter im wesentlichen gleichförmigem Abstand angeordnet sind.

10. Gelenkprothese nach Anspruch 9, dadurch gekennzeichnet, daß die sekundären Verankerungselemente (38) axial benachbarter Gruppen von sekundären Verankerungselementen in Umfangsrichtung gegeneinander versetzt sind, vorzugsweise unter gleicher Winkelteilung angeordnet und um eine halbe solche Teilung gegeneinander versetzt sind.

11. Gelenkprothese nach einem der Ansprüche 1-10, dadurch gekennzeichnet, daß die Verankerungselemente (38) im unbelasteten Zustand über eine Sollbegrenzungsfläche (24, 26) für eine Knochenausnehmung überstehen.

12. Gelenkprothese nach Anspruch 11, dadurch gekennzeichnet, daß die Verankerungselemente (38) in ihren über die Sollbegrenzungsfläche (24, 26) überstehenden Abschnitten, eine ballige Kontaktfläche vorgeben.

13. Gelenkprothese nach Anspruch 12, dadurch gekennzeichnet, daß die ballige Kontaktfläche durch an die Verankerungselemente (38) angeformte, über die Verankerungsscheiben (42) radial überstehende Kontaktkörper (45) gebildet ist.

14. Gelenkprothese nach Anspruch 12 in Verbindung mit Anspruch 8, dadurch gekennzeichnet, daß die Kontaktfläche durch Abschnitte der kreisbogenförmigen sekundären Verankerungselemente (38) gebildet ist, welche keine radial nach außen überstehenden Abschnitte von Verankerungsscheiben (42) aufweisen.

15. Gelenkprothese nach Anspruch 14, dadurch gekennzeichnet, daß die die Kontaktfläche bildenden Abschnitte der kreisbogenförmigen sekundären Verankerungselemente (38) radial von der Sollbegrenzungsfläche (24, 26) nach innen verlaufende halbkreisförmige oder exzentrische kreisförmige Verankerungsscheiben (42) tragen.

16. Gelenkprothese nach einem der Ansprüche 1-15, dadurch gekennzeichnet, daß die Teilflächen, über welche die Verankerungsscheiben (42) mit gegen sie nach Implantation gegengewachsener Spongiosa in Berührung stehen, so gewählt sind, daß bei kurzfristigen Arbeitsbelastungen der Spongiosa etwa 120%-140% der Druckfließgrenze der Spongiosa erreicht wird, während die lokalen Belastungen der Spongiosa bei Langzeitbelastung bei etwa 60% bis etwa 80% der Druckfließgrenze bleiben.

17. Gelenkprothese nach Anspruch 16, dadurch gekennzeichnet, daß die Gesamtoberfläche der Verankerungselemente (38) etwa 130% bis etwa 270% der Wandfläche der Aufnahme (24, 26) beträgt, die zur Impantierung des betrachteten Prothesenteiles aus einem Knochen zunächst ausgeräumt werden muß.

18. Gelenkprothese nach einem der Ansprüche 1-17, dadurch gekennzeichnet, daß die Verankerungselemente (38) etwa folgende Abmessung haben: Kerndurchmesser etwa 0,8-2,0 mm, Durchmesser der Verankerungsscheiben (42) etwa um 0,6-1,2 mm größer als der Kerndurchmesser; axiale Abmessung der Verankerungsscheiben etwa 0,5-1,0 mm, axialer Abstand der Verankerungsscheiben(42) ca. 2,0-4,0 mm.

19. Gelenkprothese nach einem der Ansprüche 1-18, dadurch gekennzeichnet, daß die Gelenkelemente (20, 22) aus einem gewebeverträglichen thermoplastischen Kunststoff, insbesondere Polyäthylen, hergestellt sind, und auf ihrer Rückseite jeweils mindestens einen Befestigungszapfen (32, 34) tragen, der durch Formschluß oder thermisches Nieten an einer zugeordneten Öffnung (30) der Tragplatte (28) festgelegt ist.

20. Gelenkprothese nach einem der Ansprüche 1-19, dadurch gekennzeichnet, daß ein erstes Gelenkelement (20) eine sphärische Gelenkpfanne (48) und ein zweites Gelenkelement eine sphärische Gelenkkugel (52) aufweist und daß der Gelenkkugel (52) eine Anschlagscheibe (54) zugeordnet ist, welche mit dem ersten Gelenkelement (20) bei Erreichen einer vorgegebenen Verkippung der Gelenkelemente (20, 22) gegeneinander in Eingriff kommt.

21. Gelenkprothese nach Anspruch 20, dadurch gekennzeichnet, daß die Anschlagscheibe (54) zumindest in ihrem Randabschnitt radial frei auskragend und elastisch verformbar ist.

22. Gelenkprothese nach Anspruch 21, dadurch gekennzeichnet, daß zumindest ein Teil des Randabschnittes der Anschlagscheibe (54) mit radialen Schlitzen (56) versehen ist.

23. Gelenkprothese nach einem der Ansprüche 1-19, dadurch gekennzeichnet, daß ein erstes Gelenkelement (20) eine zylindrische Gelenkpfanne (48) und ein zweites Gelenkelement (22) eine zylindrische Gelenkkugel (52) aufweist und daß die Gelenkpfanne (48) über eine Stift/Langloch-Verbindung (64, 66) mit der Gelenkkugel (52) verbunden ist.

24. Gelenkprothese nach Anspruch 23, dadurch gekennzeichnet, daß die Stift/Langlochverbindung (64, 66) in zur Hauptbewegungsebene des im wesentlichen einachsigen Zylindergelenkes senkrechter Richtung Spiel aufweist.

25. Gelenkprothese nach einem der Ansprüche 1-19, dadurch gekennzeichnet, daß das eine Prothesenteil (14) ein zylindrisches Wälzelement (78) und das andere Prothesenteil (12) eine im wesentlichen ebene Wälzfläche (80) aufweist und Führungsmittel (84-90) vorgesehen sind, welche zulassen: ein Abheben des Wälzelementes (78) von der Wälzfläche (80) bei Zugbelastung des Gelenkes, ein Abrollen des Wälzelementes (78) auf der Wälzfläche (80) unter Druckbelastung und ein Sichdurchdrehen des Wälzelementes (78) über der Wälzfläche (80) bei verglichen mit dem ausgeübten Drehmoment geringer Zugbelastung des Gelenkes, wobei die Führungsmittel aufweisen: einen Führungsstift (90), der parallel zur Wälzfläche (80) verläuft und vom einen Prothesenteil (14) getragen ist, sowie einen konturierten Führungsschlitz (86, 88), der vom anderen Prothesenteil (12) getragen ist, in einer zur Wälzfläche (80) senkrechten Ebene liegt und einen der Wälzfläche (80) benachbarten Schlitzabschnitt (86) hat, in welchem sich der Führungsstift (90) unter Spiel bei Zugbelastung des Gelenkes bewegen kann, und einen gemäß einer Abrollbewegung erweiterten Schlitzabschnitt (88) hat, in welchem sich der Führungsstift (90) beim Abrollen des Wälzelementes (78) auf der Wälzfläche (80) unter Spiel bewegt.

## Claims

1. Joint prosthesis for small joints such as finger or toe joints with two prosthesis parts (12, 14) which each comprise a fixing unit (16, 18) and a joint element (20, 22) carried thereby, wherein the fixing units (16, 18) each comprise a carrying plate (28) and at least one fixing element (38) which carries a plurality of axially succeeding radial fixing discs (42), characterised in that the fixing elements (38) together have a substantially conical external contour and are elastically and/or plastically deformable at least in one end region so they automatically adapt themselves to the bone geometry when inserted into the end of a bone.

2. Joint prosthesis according to claim 1, characterised in that the fixing elements (38) are substantially rod-shaped and are each arranged individually on the carrying plate (28).

3. Joint prosthesis according to claim 2, characterised in that the fixing elements (38) are connected in the vicinity of the carrying plate (28) by reinforcing elements (44) which also carry a plurality of axially succeeding radial fixing discs (42).

4. Joint prosthesis according to claim 1 or 2, characterised in that a single fixing element (38) is arranged substantially centrally on the carrying plate (28), in that the fixing discs (42), starting from a diameter comparable to their diameter in the vicinity of the carrying plate (28), have a diameter which decreases progressively toward the free end of the fixing element (38) and in that the fixing discs (42) have axial slits (58) in one end region of the fixing element (38).

5. Joint prosthesis according to claim 4, characterised in that the fixing discs (42) are designed as grids.

6. Joint prosthesis according to claim 4 or 5, characterised in that the radially external portions of the fixing discs 42 are provided with slits (76) and are radially deformable.

7. Joint prosthesis according to claims 1, 2 or 4, characterised in that secondary fixing elements (38) are arranged on a main fixing element (68) fastened substantially centrally on the carrying plate (28), are elastically and/or plastically deformable at least in their end portions and each carry a plurality of axially spaced fixing discs (42) having a diameter smaller than the diameter of the carrying plate (28).

8. Joint prosthesis according to claim 7, characterised in that the secondary fixing elements (38) are each substantially arcuate, their peripheral span being greater than 180° but preferably smaller than 225° and the open side of the arc being directed toward the carrying plate (28).

9. Joint prosthesis according to claim 7 or 8, characterised in that the secondary fixing elements (38) are arranged in axially spaced groups, the secondary fixing elements (38) of one group located in a common radial plane being arranged peripherally with substantially uniform spacing.

10. Joint prosthesis according to claim 9, characterised in that the secondary fixing elements (38) of axially adjacent groups of secondary fixing elements are peripherally mutually offset, are preferably arranged with identical angular pitch and are mutually offset by half such a pitch.

11. Joint prosthesis according to one of claims 1 to 10, characterised in that the fixing elements (38) in the unloaded state project beyond a set limiting face (24, 26) for a bone recess.

12. Joint prosthesis according to claim 11, characterised in that the fixing elements (38) provide a convex contact face in their portions projecting beyond the set limiting face (24, 26).

13. Joint prosthesis according to claim 12, characterised in that the convex contact face is formed by contact members (45) shaped on the fixing elements (38) and projecting radially beyond the fixing discs (42).

14. Joint prosthesis according to claim 12 in conjunction with claim 8, characterised in that the contact face is formed by portions of the arcuate secondary fixing elements (38) having no radially outwardly projecting portions of fixing discs (42).

15. Joint prosthesis according to claim 14, characterised in that the portions of the arcuate secondary fixing elements (38) forming the contact face carry semi-circular or eccentric circular fixing discs (42) which extend radially inwards from the set limiting face (24, 26).

16. Joint prosthesis according to one of claims 1 to 15, characterised in that the partial faces by means of which the fixing discs (42) make contact with the spongy tissue, which has grown against them after implantation, are selected such that 120 to 140% of the liquid limit of the spongy tissue is attained during short-term loading of the spongy tissue whereas the local loading of the spongy tissue remains at about 60% to 80% of the liquid limit during long-term loading.

17. Joint prosthesis according to claim 16, characterised in that the total surface area of the fixing elements (38) is about 130% to about 270% of the wall area of the cavity (24, 26) which initially has to be cleared from a bone for implantation of the prosthesis part under consideration.

18. Joint prosthesis according to one of claims 1 to 17, characterised in that the fixing elements (38) have roughly the following dimensions: core diameter about 0.8 to 2.0 mm, diameter of the fixing discs (42) about 0.6 to 1.2 mm greater than the core diameter, axial dimension of the fixing discs about 0.5 to 1.0 mm, axial spacing of the fixing discs (42) about 2.0 to 4.0 mm.

19. Joint prosthesis according to one of claims 1 to 18, characterised in that the joint elements (20, 22) are produced from a thermoplastic polymer, in particular polyethylene, which is compatible with tissue and, on their rear, each carry at least one fastening peg (32, 34) which is secured by interlocking or thermal riveting on an associated aperture (30) in the carrying plate (28).

20. Joint prosthesis according to one of claims 1 to 19, characterised in that a first joint element (20) has a spherical joint socket (48) and a second joint element has a spherical joint ball (52) and in that the joint ball (52) is allocated a stop disc (54) which engages with the first joint element (20) when predetermined tilting of the joint elements (20, 22) is attained.

21. Joint prosthesis according to claim 20, characterised in that the stop disc (54) protrudes radially freely at least in its edge portion and is elastically deformable.

22. Joint prosthesis according to claim 21, characterised in that at least a part of the edge portion of the stop disc (54) is provided with radial slits (56).

23. Joint prosthesis according to one of claims 1 to 19, characterised in that a first joint element (20) has a cylindrical joint socket (48) and a second joint element (22) has a cylindrical joint ball (52) and in that the joint socket (48) is connected to the joint ball (52) via a pin/slot connection (64, 66).

24. Joint prosthesis according to claim 23, characterised in that the pin/slot connection (64, 66) has play in the direction perpendicular to the main plane of movement of the substantially uniaxial cylinder joint.

25. Joint prosthesis according to one of claims 1 to 19, characterised in that one prosthesis part (14) has a cylindrical rolling element (78) and the other prosthesis part (12) a substantially plane rolling face (80) and guide means (84 to 90) are provided which allow: lifting of the rolling element (78) from the rolling face (80) with tensile loading of the joint, rolling of the rolling element (78) on the rolling face (80) with pressure loading and spinning of the rolling element (78) over the rolling face (80) when the tensile loading of the joint is lower than the torque exerted, the guide means comprising: a guide pin (90) which extends parallel to the rolling face (80) and is carried by a prosthesis part (14) as well as a contoured guide slit (86, 88) which is carried by the other prosthesis part (12), lies in a plane perpendicular to the rolling face (80) and has a slit portion (86) which is adjacent to the rolling face (80) and in which the guide pin (90) can move with play with tensile loading of the joint and has a slit portion (88) which is enlarged according to a rolling movement and in which the guide pin (90) moves with play when the rolling element (78) rolls on the rolling face (80).

## Revendications

1. Prothèse articulaire pour petites articulations telles que des articulations des doigts ou des orteils, comprenant deux parties (12, 14) qui présentent, à chaque fois, une unité d'ancrage (16, 18) et un élément d'articulation (20, 22) porté par cette dernière, les unités d'ancrage (16, 18) étant respectivement munies d'une plaque de support (28) et d'au moins un élément d'ancrage (38) qui porte une pluralité de disques radiaux d'ancrage (42) se succédant dans la direction axiale, caractérisée par le fait que les éléments d'ancrage (38) possèdent conjointement un profil extérieur pour l'essentiel conique, et sont déformables élastiquement et/ou plastiquement dans au moins une région extrême, de sorte qu'ils s'adaptent automatiquement à la géométrie osseuse lors de l'insertion dans l'extrémité d'un os.

2. Prothèse articulaire selon la revendication 1, caractérisée par le fait que les éléments d'ancrage (38) revêtent pour l'essentiel la forme de barreaux et sont, à chaque fois, ménagés individuellement sur la plaque de support (28).

3. Prothèse articulaire selon la revendication 2, caractérisée par le fait que les éléments d'ancrage (38) sont reliés, au voisinage de la plaque de support (28), par des éléments de rigidification (44) portant, pareillement, une pluralité de disques radiaux d'ancrage (42) se succédant dans la direction axiale.

4. Prothèse articulaire selon la revendication 1 ou 2, caractérisée par le fait qu'un unique élément d'ancrage (38) est ménagé, pour l'essentiel centralement, sur la plaque de support (28) ; par le fait que les disques d'ancrage (42) présentent un diamètre s'amenuisant progressivement en direction de l'extrémité libre de l'élément d'ancrage (38), à partir d'un diamètre comparable au diamètre de la plaque de support (28) au voisinage de cette dernière ; et par le fait que les disques d'ancrage (42) comportent des fentes axiales (58) dans une région extrême de l'élément d'ancrage (38).

5. Prothèse articulaire selon la revendication 4, caractérisée par le fait que les disques d'ancrage (42) sont réalisés sous la forme de quadrillages.

6. Prothèse articulaire selon la revendication 4 ou 5, caractérisée par le fait que les régions des disques d'ancrage (42), qui sont situées à l'extérieur dans le sens radial, sont pourvues de fentes (76) et sont déformables radialement.

7. Prothèse articulaire selon l'une des revendications 1, 2 ou 4, caractérisée par le fait que des éléments d'ancrage secondaires (38), ménagés sur un élément d'ancrage principal (68) fixé pour l'essentiel centralement à la plaque de support (28), sont déformables élastiquement et/ou plastiquement au moins dans leurs régions extrêmes et portent, à chaque fois, une pluralité de disques d'ancrage (42) espacés axialement et présentant un petit diamètre comparativement au diamètre de ladite plaque de support (28).

8. Prothèse articulaire selon la revendication 7, caractérisée par le fait que les éléments d'ancrage secondaires (38) sont, pour l'essentiel, respectivement configurés en des arcs de cercle, leur étendue périphérique mesurant plus de 180°, mais toutefois moins de 225°, de préférence, et le côté ouvert de l'arc de cercle étant tourné vers la plaque de support (28).

9. Prothèse articulaire selon la revendication 7 ou 8, caractérisée par le fait que les éléments d'ancrage secondaires (38) sont agencés en des groupes espacés axialement, les éléments d'ancrage secondaires (38) d'un groupe, situés dans un plan radial commun, étant disposés selon un espacement pour l'essentiel uniforme dans le sens périphérique.

10. Prothèse articulaire selon la revendication 9, caractérisée par le fait que les éléments d'ancrage secondaires (38), faisant partie de groupes d'éléments d'ancrage secondaires voisins dans le sens axial, sont décalés les uns des autres dans le sens périphérique et sont, de préférence, disposés selon une répartition angulaire identique, et mutuellement décalés de la moitié d'une telle répartition.

11. Prothèse articulaire selon l'une des revendications 1-10, caractérisée par le fait que les éléments d'ancrage (38) dépassent, à l'état non contraint, au-delà d'une surface de délimitation de consigne (24, 26) destinée à un évidement osseux.

12. Prothèse articulaire selon la revendication 11, caractérisée par le fait que les éléments d'ancrage (38) prédéfinissent une surface bombée de contact dans leurs régions saillant au-delà de la surface de délimitation de consigne (24, 26).

13. Prothèse articulaire selon la revendication 12, caractérisée par le fait que la surface bombée de contact est formée par des corps de contact (45) venus solidairement de moulage sur les éléments d'ancrage (38) et faisant saillie, dans le sens radial, au-delà des disques d'ancrage (42).

14. Prothèse articulaire selon la revendication 12 rattachée à la revendication 8, caractérisée par le fait que la surface de contact est formée par des régions des éléments d'ancrage secondaires (38) en arc de cercle qui sont dépourvues de segments de disques d'ancrage (42) faisant saillie radialement vers l'extérieur.

15. Prothèse articulaire selon la revendication 14, caractérisée par le fait que les régions des éléments d'ancrage secondaires (38) en arc de cercle, qui forment la surface de contact, portent des disques circulaires d'ancrage (42), semi-circulaires ou excentrés, s'étendant radialement vers l'intérieur à partir de la surface de délimitation de consigne (24, 26).

16. Prothèse articulaire selon l'une des revendications 1-15, caractérisée par le fait que les surfaces partielles, par lesquelles les disques d'ancrage (42) sont en contact avec de la substance spongieuse s'étant développée contre lesdits disques après implantation, sont choisies de telle sorte qu'environ 120 %-140 % de la limite de fluage sous pression de la substance spongieuse soient atteints en cas de contraintes opératoires de courte durée de ladite substance spongieuse, tandis que les contraintes locales de la substance spongieuse demeurent d'environ 60 % à environ 80 % de la limite de fluage sous pression en cas de contrainte de longue durée.

17. Prothèse articulaire selon la revendication 16, caractérisée par le fait que la surface totale des éléments d'ancrage (38) représente d'environ 130 % à environ 270 % de la surface de la paroi du logement (24, 26) devant tout d'abord être pratiqué, par dépouille d'un os, en vue de l'implantation de la partie de prothèse considérée.

18. Prothèse articulaire selon l'une des revendications 1-17, caractérisée par le fait que les éléments d'ancrage (38) présentent approximativement le dimensionnement suivant : diamètre de noyau d'environ 0,8-2,0 mm ; diamètre des disques d'ancrage (42) excédant d'environ 0,6-1,2 mm le diamètre du noyau ; dimension axiale des disques d'ancrage d'environ 0,5-1,0 mm ; espacement axial des disques d'ancrage (42) d'environ 2,0-4,0 mm.

19. Prothèse articulaire selon l'une des revendications 1-18, caractérisée par le fait que les éléments d'articulation (20, 22) sont fabriqués en une matière thermoplastique compatible avec les tissus, notamment du polyéthylène, et portent respectivement, sur leur face postérieure, au moins un tenon de fixation (32, 34) verrouillé à demeure, par concordance de formes ou par rivetage thermique, sur un orifice associé (30) de la plaque de support (28).

20. Prothèse articulaire selon l'une des revendications 1-19, caractérisée par le fait qu'un premier élément d'articulation (20) présente une cuvette sphérique d'articulation (48) et un second élément d'articulation possède une rotule sphérique d'articulation (52) ; et par le fait qu'un disque de butée (54), associé à la rotule d'articulation (52), vient en prise avec le premier élément d'articulation (20) lorsqu'est atteint un basculement préétabli desdits éléments d'articulation (20, 22) l'un par rapport à l'autre.

21. Prothèse articulaire selon la revendication 20, caractérisée par le fait que le disque de butée (54) fait librement saillie dans le sens radial et est élastiquement déformable, au moins dans sa région marginale.

22. Prothèse articulaire selon la revendication 21, caractérisée par le fait qu'au moins une partie de la région marginale du disque de butée (54) est pourvue de fentes radiales (56).

23. Prothèse articulaire selon l'une des revendications 1-19, caractérisée par le fait qu'un premier élément d'articulation (20) présente une cuvette cylindrique d'articulation (48) et un second élément d'articulation (22) possède une rotule cylindrique d'articulation (52) ; et par le fait que la cuvette d'articulation (48) est reliée à la rotule d'articulation (52) par l'intermédiaire d'une solidarisation (64, 66) par broche/trou oblong.

24. Prothèse articulaire selon la revendication 23, caractérisée par le fait que la solidarisation (64, 66) par broche/trou oblong présente un jeu dans une direction perpendiculaire au plan de mouvement principal de l'articulation cylindrique munie, pour l'essentiel, d'un axe unique.

25. Prothèse articulaire selon l'une des revendications 1-19, caractérisée par le fait que l'une (14) des parties de la prothèse présente un élément cylindrique de roulement (78) et l'autre partie (12) de la prothèse comporte une surface de roulement (80) pour l'essentiel plane, et l'on a prévu des moyens de guidage (84-90) autorisant : un soulèvement de l'élément de roulement (78) à l'écart de la surface de roulement (80) en cas de contrainte de traction de l'articulation, un roulement de l'élément de roulement (78) sur la surface de roulement (80) en cas de contrainte de pression, et une rotation continue de l'élément de roulement (78), au-dessus de la surface de roulement (80), dans le cas où l'articulation est soumise à une contrainte de traction faible comparativement au couple de rotation imposé ; les moyens de guidage comprenant : un téton de guidage (90) s'étendant parallèlement à la surface de roulement (80) et porté par l'une (14) des parties de la prothèse, ainsi qu'une fente ininterrompue de guidage (86, 88) qui est portée par l'autre partie (12) de la prothèse, se trouve dans un plan perpendiculaire à la surface de roulement (80), comporte un segment (86) voisin de ladite surface de roulement (80), dans lequel le téton de guidage (90) peut se mouvoir avec jeu lorsque l'articulation subit une contrainte de traction, et présente un segment (88) élargi en conformité avec un mouvement de roulement, et dans lequel ledit téton de guidage (90) se meut avec jeu lorsque l'élément de roulement (78) roule sur ladite surface de roulement (80).
